# EUROPEAN PATENT APPLICATION

(11) **EP 4 059 440 A1**
(43) Date of publication of application: **21.09.2022**
(21) Application number: 21162705.4
(22) Date of filing: 15.03.2021
(51) Int. Cl.: A61B 8/08, A61B 8/00

(54) **ULTRASOUND SYSTEM**

(71) Applicant: Pulsify Medical, 3001 Leuven (BE)
(72) Inventor: MORAES, Rodrigo Bastos, 3001 Leuven (BE); STOFFELS, Steve, 3001 Leuven (BE); VAN DER WAAL, Erwin, 3001 Leuven (BE)
(74) Representative: DTS Patent- und Rechtsanwälte Schnekenbühl und Partner mbB

(57) **Abstract**

The invention relates to an ultrasound system (1), in particular for cardiac monitoring, comprising a transducer array (3) with a plurality of transducer array elements (3a), a control unit (40), which is coupled to the transducer array (3), for controlling the transducer array (3), and a data processing unit (41) for receiving data from the transducer array (3) and processing the received data. Therein, the ultrasound system (1) has at least two operating modes, namely a sleep mode and a measuring mode. The control unit is configured, when the sleep mode is activated, to have a low frequency system clock running to a predetermined sleep frequency, and, when the measuring mode is activated, a high frequency system clock is set to a measuring frequency.

## Description

The present invention relates to an ultrasound system, in particular for cardiac monitoring.

Wearable ultrasound monitor systems monitor cardiac function over time. The ultrasound monitor can be body worn in a patch type format.

US 2016/0136462 A1 discloses a wearable ultrasound device and method of using the device including a power controller with a power source and at least one integrated circuit that delivers electrical power to an applicator. The applicator is electrically coupled to the power controller and a surface of the applicator transmits ultrasound to a wearer for a given duration. The applicator includes radio frequency (RF) drive electronics, an ultrasound transducer coupled to the drive electronics, a monitoring apparatus that includes a thermal cutoff coupled to the drive electronics.

WO 2018/102828 A1 discloses an ultrasound coupling patch for use with ultrasound transducers, and more particularly to ultrasound coupling patches having a gel capture feature.

WO 2019/162485 A1 discloses a method and an apparatus for performing ultrasound measurements of a propagation medium containing non uniformities, with a transmitter/transducer with a plurality of transmitting elements for emitting ultrasound excitation pressure waves.

In ultrasound systems with a large number of ultrasound transducer elements, e.g., when a transducer array with 10.000 or 100.000 elements is used, individually sampling every single transducer element leads to massive amounts of data. The system needs accordingly high capabilities for the throughput and the processing of this data. A high amount of power is required to drive and read such an array and all its individual transducer elements.

Power consumption is a crucial factor for operating the ultrasound system, especially when the system is configured for long-term monitoring. It is even more important for a portable ultrasound device, which may comprise or be connected to a portable power source with a limited capacity.

It is the problem of the invention to provide an ultrasound system that allows efficient use of available power resources, while still offering high flexibility and optimum data quality.

This problem is solved by an ultrasound system according to claim 1 of the attached set of claims. Further advantageous embodiments are given in the dependent claims.

The ultrasound system, in particular for cardiac monitoring, comprises a transducer array with a plurality of transducer array elements, a control unit, which is coupled to the transducer array, for controlling the transducer array, and a data processing unit for receiving data from the transducer array and processing the received data. The ultrasound system has at least two operating modes, namely a sleep mode and a measuring mode. The control unit is configured, when the sleep mode is activated, to have a low frequency system clock running to a predetermined sleep frequency, and, when the measuring mode is activated, to set a high frequency system clock to a measuring frequency.

In particular, the ultrasound system can be in general an ultrasound system as disclosed by WO 2019/162485 A1. The further disclosed features can be added for example to the system as disclosed in WO 2019/162485 A1.

Furthermore, the system may have a third operating mode, namely a power-down mode, in which all elements of the system are switched off.

Additionally, the control unit is configured to only have the low frequency system clock running, when the sleep mode is activated. Herein, the high frequency system clock may be deactivated during sleep mode.

Thus, the power consumption of the system can advantageously be reduced. Also, it is possible to switch between appropriate operating modes, if the context makes this necessary.

The invention is based on the idea that the power consumption of the ultrasound system is greatly influenced by the activity of a high frequency clock. This is due to the need for a very fast control of transducer elements, typically in the range of 20-200 MHz, which is used for timing the generation of ultrasound pulses, receiving ultrasound signals and/or processing and analyzing the received data. The low frequency keeps those parts of the system running, which are important for the sleep mode operation. In particular, such parts of the system are running, which are needed to activate the measuring mode, i.e., to perform a defined power-up sequence. When the measuring-mode is activated, the high frequency system clock is activated in a power-up sequence, so measurements can be performed. On the other hand, the ultrasound device's operation is not inhibited or only minimally impacted.

The system may be configured for cardiac monitoring, which may for example comprise monitoring of cardiac activity and/or cardiac properties of a patient. Monitoring cardiac activity may comprise monitoring of parameters such as cardiac rhythm and/or cardiac output.

The system has at least one transducer array, i.e., a device or unit comprising a plurality of transducer array elements. In particular, the transducer array may have a chip design. The transducer array elements are units, which may be used as an actuator for generating an ultrasound pulse or signal and/or as a receive unit for detecting incoming ultrasound waves. Generally speaking, one transducer array element may consist of one or more transducer sub-elements. Such a sub-element can be a single transducer only, which can be a piezoelectric micromachined ultrasonic transducer (pMUT) component or a capacitive micromachined ultrasonic transducer (cMUT) component.

In an embodiment, a transducer array element may for example consist of four, nine, sixteen transducer sub-elements, which may be arranged in either a 2x2, 3x3, 4x4 grid or in another suitable fashion. Also non square configurations can be used e.g. for 6 sub-elements in a 2x3 configuration. In further embodiments, other numbers of transducer sub-elements in one transducer array element can be provided, and the transducer sub-elements of one transducer array element may be arranged in a grid or in another arrangement, for example similar to a crystal lattice arrangement. The sub-elements may be connected either in a series and/or parallel connection and provide a common input and output such that they can be operated as a single functional unit.

For example, one ultrasound transducer element may comprise one or more piezoelectric transducer sub-element(s), such as piezoelectric micromachined ultrasonic transducers (pMUT).

Different techniques may be used to manufacture transducer elements and/or sub-elements. For example, cMUT units may be fabricated using a polymer-based technique, such as described by Gerardo et al. in "Fabrication and testing of polymer-based capacitive micromachined ultrasound transducers for medical imaging", (Microsystems & Nanoengineering (2018) 4:19). Other examples are WO 2014/134301 A1, US 8,345,512 B2 and US 2005/0146247 A1.

In order to reduce the complexity of the system, several transducer elements may be combined into a functional unit and treated as a single "element", i.e., the combined transducer array elements are driven at that same time as if they are a single unit.

The transducer array elements in the transducer array can be connected in series or parallel, such that they can be driven optimally. For example, in a 2D array configuration the transducer elements could be connected in parallel in the direction of the rows or columns of the array to create a row/column based architecture.

In particular, the transducer array elements may be configured such that they are individually controllable.

In a possible embodiment of the system, the transducer array is flexible and/or stretchable. Thus, the system can advantageously adjust its geometry to a probe or sample surface, e.g., a patient's body.

For example, the transducer array elements may be supported by a flexible substrate such that they are arranged on a flat or curved plane. The substrate may, e.g., comprise a polymer material.

In a further possible embodiment of the system, the transducer array is integrated into an patch for fixing the transducer array to a patient's body by means of either an adhesive or a strap. The system can then be easily used, e.g., for monitoring a patient's body functions, such as cardiac functions, especially for long-term monitoring.

The transducer array may be integrated into the patch by providing it with an adhesive area, which is suited for attaching the patch to a patient's body surface. Also, a textile or other flexible material may be provided with an adhesive material and used to fix the transducer array to the body surface. In particular, the patch may be configured such that it secures the transducer array in close proximity to the body surface.

Also, the ultrasound system comprises a control unit. The control unit may comprise several components, which may be structurally integrated with each other, for example in a common casing or envelope structure, or which may be implemented as structurally separated components, which may be couple to each other.

The transducer array elements are configured to generate and/or receive ultrasound, in particular ultrasound pulses or signals, based on a control signal. To provide the respective control signals for the transducer array, the control unit may comprise a send module and a receive module, which may be implemented in a driver module for the transducer array and/or for transistors, which may control the transducer array elements.

The control unit may be configured to perform different tasks such as, but not limited to, the following examples: The control unit may be configured to perform digital signal processing and/or filtering. Also, the control unit may be configured to perform operations related to storing and/or retrieving information, in particular in a memory module, such as a random access memory (RAM) module. Also, the control unit may be configured to perform operations for driving an external interface, such as an interface to a memory module, a transceiver unit, e.g., via a USB interface or a wireless interface, and/or receiving control signals from other units. The control unit may also perform operations for controlling the transducer array, e.g., a transistor linked to a transducer array element, such as a TFT element, or a matrix of such transistors, e.g., in a TFT matrix. Also, the control unit may be configured to control a transducer element or transistor, to generate delays for generating and/or sensing ultrasound pulses and signals, to define generated ultrasound pulses, beamforming operations, and/or to control power management. Each of said functions may be carried out and/or implemented in an ASIC design individually or in combination. Also, these functions may be implemented as modules of the control unit.

The control unit may comprise a switch, e.g. a transistor or thin-film transistor (TFT), which can be configured to control a transducer array element. In particular, an array of switches/transistors/thin-film transistors may be provided, which is coupled to the transducer array.

A TFT array (or more generally speaking the switching module) of the system is used to control the transducer array. The TFT array or the switching module can be coupled to the transducer array and especially it may be coupled to the input ports of the transducer elements.

In particular, the switch array (e.g. TFT array) may be structurally integrated with the transducer array in one component namely the sensor array. This integration can be done, e.g., in a common casing, envelope structure or monolithically.

The control unit may consist of or comprise one or several microprocessors. Also, the control unit may comprise an application-specific integrated circuit (ASIC) design. The control unit, in particular a microprocessor of the control unit, may be fully or partially integrated into a printed circuit board (PCB). The control unit may also comprise of control circuitry integrated on the panel for the TFT array, this control circuitry will work in conjunction with but as a separate functional unit compared to the TFT switch elements, which enable the selection of the transducer elements. The PCB might be rigid, flexible, stretchable or a hybrid combination thereof.

The system may comprise a field programmable gate array (FPGA) and/or a digital application-specific integrated circuit (ASIC), configured such that one or more control algorithms for the transducer array are carried out by these components. The FPGA and/or ASIC may be part of the control unit.

Additionally, the system further comprises a beam former module. Herein, the beam former module is configured to apply a time delay to at least one transducer array element or a plurality of transducer array elements. Preferably multiple delays are applied to different sets of transducers array elements. This allows advantageously easy control for high-resolution measurements, beam focusing and/or beam steering. In particular, the time delays may be applied, when the transducer array element or the plurality of transducer array elements is used for sending ultrasound pulses and/or receiving ultrasound signals.

The time delay for a transducer array element or a plurality of transducer array elements may be defined relative to controlling other transducer array elements.

Also, the beam former module and/or another unit of the system may be utilized to determine the delay. In particular, the delay is determined for each transducer array element or for a plurality of transducer array elements, to which the determined delay is to be applied.

In particular, the control unit may comprise an ASIC for implementing a beam former control, and/or a design for a signal processing algorithm, e.g., for applying spatial and/or temporal sparsity conditions for sending ultrasound pulses and/or for collecting or providing data (related to the measurements performed by the ultrasound device and the ultrasound pulses and signals). In particular, the beam former control can for example comprise an electronic delay and sum circuit that can apply different delays to a certain number of transducer array elements, for example 8 to 32, e.g. 14, 15, 16, 17 or 18 transducer array elements or functional units comprising several connected transducer array elements (e.g. 14, 15, 16, 17 or 18).. It may sum the output of these channels into one single output value. In particular, by applying specific delays to each functional unit of transducer array elements, an ultrasound pulse may be generated in a coordinated fashion such that a specific profile for the ultrasound beam is obtained. Also, elements receiving ultrasound signals may be controlled by a beam former in a coordinated way to provide targeted measurements inside a sample volume.

To send the ultrasound pulses a voltage pulse is applied to the inputs of the sensor array, each sensor input connects to at least one but preferably multiple transducer elements, for example 8 to 32 inputs, such as e.g. 14, 15, 16, 17 or 18 inputs can be provided to the sensor array whereby each input connects to e.g. 14, 15, 16, 17 or 18 parallel connected transducer elements. Applying the pulses to the sensor input channels with different delays allows to shape the ultrasound beam, e.g. steering it and/or focusing it, providing a divergent or planar wave.

The data processing unit may be implemented in different ways. It may for example comprise a microprocessor. In particular, it may comprise an ASIC design, it may also comprise an FPGA or DSP. Also, the data processing unit and the control unit may be implemented as software modules that are run, at least partially, on a common computational unit. For example, a processing unit may read from a memory and execute instructions for performing operations to implement the data processing unit and/or control unit.

Also, the data processing unit may comprise one or several analog-digital converters (ADC) and/or digital-analog converters (DAC). These converters are configured to link analog and digital channels to each other and allow both digital data collection and treatment, and treating analog control and detection signals. A time gain controller may be implemented using a DAC element and/or a different architecture.

A complexity reduction module may be implemented as software module, comprising instructions for computational operations that implement the function of complexity reduction. This software module may in particular be implemented as a module of the control unit. Specifically, the complexity reduction module may be comprised, at least partially, by the control unit and/or by the data processing unit.

The complexity reduction module implements functions to reduce data and sensor complexity and/or quantity. Such functions may impact the generation and/or the detection of ultrasound pulses and signals by the transducer array, or they may influence the data acquisition and/or treatment.

The coupling between the transducer array and the control unit may comprise a wire connection, in particular an interface for data and/or power transmission between the transducer array and the control unit. In particular, the control unit and/or a part of the control unit may be arranged on the sensor array or may be integrated with the sensor array into one common part. The sensor array can be embodied especially by means of the combination of a TFT array and a transducer array.

It is possible that there is a wire connection and/or a wireless interface provided, if the control unit controls the sensor array. If a wireless sensor array is implemented, the control unit may be arranged on the sensor array and/or integrated into one component with the sensor array.

Furthermore, if the sensor array is connected to another element by a wire, at least part of the controlling of the sensor array may be implemented on the sensor array and/or integrated into one component with the sensor array. Also, an additional control unit may be provided, e.g., in a separate PC or control unit, when there is access through wires to the sensor array. A sensor array is for example a combination of a transducer array and a TFT array. Parts of the controller can be integrated on a TFT array by an integrated circuit design or by bonding ICs to the TFT.

In a further possible embodiment, the ultrasound system further comprises a power source unit for providing energy to the transducer array and/or the control unit. The system can thus be advantageously used in a very flexible manner, in particular when a portable power source is provided. For example, the power source may comprise a battery, which is integrated in a portable device as a component of the system.

Rather than operating the transducer array as a single whole and using each element as an individual unit, a sub-array scanning architecture may be used, i.e., groups of transducer array elements are combined to functional units. In particular, neighboring elements are combined.

In particular, a control algorithm may be provided for controlling each sub-array within the transducer array. The control algorithm may control, for each sub-array, a location of the sub-array within the plurality of transducer array elements. Also, the control algorithm may control, for each sub-array, a focus depth and/or a steering angle for an ultrasound sending beam profile and/or an ultrasound receiving beam profile implemented by the sub-array of transducer array elements; such a beamforming algorithm may be implemented as a software module for a programmable unit and/or by chip design, e.g., in an ASIC.

Also, the system may be configured to generate 3D spatial information from 1D data sets collected by the transducer array, or one or more sub-arrays of the transducer array.

The system may be configured to determine a position for a sub-array within the transducer array such that it is not hindered by impediments for ultrasound beams, e.g., due to ribs of a patient close to the surface or due to air bubbles between the transducer array elements of the sub-array and a sample. To collect data in order to generate, e.g., 3D spatial information, the control unit may be configured to control, for each sub-array, the location of the sub-array, as well as the focus depth and/or a steering angle for the sub-array depending on a chosen region of interest, e.g., within a patient's body.

In order to control the profile, focusing or steering of a sub-array's ultrasound wave beam a beamforming module may be used, this allows control over the direction and depth at which can be optimally measured. The beam profile is controlled by applying delays on the individual transducer array elements or a collection of transducer array elements which provide a common input and output, i.e. a functional unit. This may be performed both for sending ultrasound pulses, thereby focusing ultrasound energy at a depth defined by a predetermined focal point and sending the ultrasound energy under a predetermined steering angle. Analog to the sending, the beam profile may be controlled for receiving ultrasound signals, e.g., in order to define a direction and a focal depth for receiving ultrasound signals, e.g., to optimally receive ultrasound signals from a predetermined location within a sample.

In an embodiment, when the sleep mode is activated, the control unit is configured to receive a wake-up signal and, subsequently, activate the measuring mode. The system can therefore, starting from a mode with low power consumption, in particular a standby mode, advantageously be quickly and easily reactivated.

The wake-up signal may be generated in different ways, in particular based on different conditions. For example, the wake-up signal may be generated by an external unit, which has a data connection to the ultrasound system via an interface module. A status register in the control unit will define in which mode, sleep or measuring, the system will be. A power-down signal will activate the power-down mode, in particular by switching off the system; a switch-off sequence may be defined and the parts of the system may be switched off according to this switch-off sequence.

For example, when the sleep mode is activated, a wake-up signal may be received when the control unit and/or the data processing unit detects any system activity such as a data query from an external unit, the lapse of a predetermined time duration in sleep mode, or triggered in another way.

In a further embodiment, the control unit may be configured to receive a sleep signal and, subsequently activate the sleep mode.

It is possible that a wake-up signal for activating the measuring mode or a sleep signal for activating the sleep mode is received, comprising activation information and the measuring or sleep mode is activated, wherein a configuration of the ultrasound system is carried out according to the activation information. For example, the activation information may comprise a parameter such as a frequency to be set in the respective activated mode. Also, other configuration information for the respective mode to be activated may be comprised by the received wake-up or sleep signal, respectively.

In a possible embodiment, when the sleep mode is activated, the standby frequency is set to a value between 25 and 50 kHz, in particular to a value of 32 kHz. The system clock is therefore advantageously set towards a value that is low enough to save considerably on power consumption, and at the same time to provide sufficient computational power to be able to re-active the system when going back to the measuring mode.

The transducer array may comprise several functional transducer array units, which may be combined in series and/or in parallel into a single element, which is driven as a single transducer array of the ultrasound system.

For example, the size and pitch of the transducer array units may be correlated to the wavelength, which is used for the ultrasound, i.e., the sound wave length (lambda). Herein, the pitch denotes the spacing from the center of one transducer array element to the neighboring transducer array element. The ultrasound waves, as they are emitted from (or received by) these transducer array elements interfere differently for different pitch values. For a pitch above half of the wavelength that is used for ultrasound, the waves constructively interfere at angles outside of the "main lobe", therefore causing so-called "grating lobes". Thus, a pitch value of lambda/2 may be used in order to avoid grating lobes. These grating lobes may negatively impact image quality when beamsteering is carried out. However a pitch value equal to lambda may also be used, if limited beam steering capabilities and some image ghosting effects are taken into account or corrected.

Different architectures may be used to address transducer array elements individually or combined in functional units. In a row column based architecture, for example, transducer array elements that are arranged in one dimension are combined and treated as a functional unit. Such elements in one dimension may be arranged along an essentially straight line. For example, instead of controlling the single elements of a transducer array individually and/or collecting signals from all transducer array elements, all elements along one axis may be combined into a single input and/or output. For example, all elements of one row or column are combined. Connecting a transducer element to a TFT element configured as a switch, allows to selectively connect a transducer element to the row or column, so that a sub-selection can be made of the active transducers along a single row or column. The pulsers and receivers for driving respectively reading the combined transducers array elements can each be connected to either the rows or columns of the array or a combination thereof; leading to possible configurations in the read-out electronics which we designate as row/row, column/column or row/column read-out schemes.

The basic concept definition for an array with the number N rows and the number M columns (if N=M, then the array is a squared array, otherwise a rectangular array) is such, that there is a controller attached to a matrix of switches, with the switch matrix consisting of a plurality of switches, such as e.g. transistor or TFT elements (the switch elements will open or close the signal path) and this controller defines, which rows are open or have to be open. Further, the controller defines or is aware of the columns and row, which are active for signal transmission and/or receiving, and the intersection of active N and active M is defining a sub-array. This way, the addressing of the transducer array elements can be done in a less complex way, as they will be addressed in the groups of transducer array elements which are called the sub-array.

It is further possible that the sleep mode of the system is optimized such that the system's power consumption is minimized, wherein only modules that are needed for activating the measuring mode are kept running. Thus, the ultrasound system can advantageously be run in the sleep mode for a very long time without consuming too much power.

Moreover, the modules, which are kept running, when the sleep mode is activated, comprise a wake-up controller module, an interrupt controller module, and/or a timer module. Thus, the necessary modules for performing a transition from sleep mode to measuring mode are kept active, while unnecessary modules are shut down.

Additionally, the control unit is configured, when activating the measuring mode after the sleep mode has been active, to perform a power-on sequence, wherein several modules of the system are subsequently activated. Thus, the system may easily switch between the different modes, especially for saving power whenever possible.

In a possible embodiment, during the power-on sequence, the first module to be activated comprises a phase-locked loop (PLL). In particular, the PLL is configured to provide a measurement frequency signal to the system clock. Thus, the system will advantageously be provided with a high frequency clock after performing the power-on-sequence.

Additionally, where individual sub-arrays are controlled as functional units for sending ultrasound pulses and/or receiving ultrasound signals, the system may for example be configured to perform ultrasound based measurements of a sample by using the sub-array as a unit cell for sending and/or receiving ultrasound pulses and/or receiving ultrasound signals. In particular, ultrasound signals are detected as reflections of a previously generated ultrasound pulses, and imaging or other data processing is performed by mapping the reflected signal and its intensity to specific areas or locations.

The transducer array elements of a sub-array can be configured such that they work as a combined functional unit, collecting 1D depth data sets by controlling the sending of an ultrasound pulse and collecting reflected ultrasound signals from a sample, e.g., from inside a patient's body.

In order to control the profile, focusing or steering of a sub-array's ultrasound wave beam a beamforming module may be used, this allows control over the direction and depth at which can be optimally measured. The beam profile is controlled by applying delays on the sensor inputs and/or outputs, these inputs/outputs connect to the individual transducer array elements or a collection of transducer array elements which provide a common input and output, i.e. a functional unit. This may be performed both for sending ultrasound pulses, thereby focusing ultrasound energy at a depth defined by a predetermined focal point and sending the ultrasound energy under a predetermined steering angle. Analog to the sending, the beam profile may be controlled for receiving ultrasound signals, e.g., in order to define a direction and a focal depth for receiving ultrasound signals, e.g., to optimally receive ultrasound signals from a predetermined location within a sample.

Also, the system may be configured to perform a sleep on sequence when the sleep-mode is activated, and the phase-locked loop may be shut down during the sleep-on sequence.

In an embodiment, the system comprises at least two transducer arrays forming a least one transducer array set; and the control unit is configured to control the transducer array set as one single virtual transducer array. By using a transducer array set for example several positions can be monitored (e.g. needed for lung monitoring) by means of one system. Further, a greater surface area can be monitored. Also, different angles for monitoring e.g. the heart and/or lung and/or chest can be established. This can also be used for a fluid detection or fluid carrying structure in a body of a patient (human or animal).

The transducer array units may be combined in series and/or in parallel into a single element, i.e., a single transducer array set, which is driven as a single transducer array of the ultrasound system.

In particular, the size and pitch of the transducer array units may be adjusted to the wavelength, which is used for the ultrasound, i.e., the sound wave length (lambda). Herein, the pitch denotes the spacing from the center of one transducer array element to the neighboring transducer array element. The ultrasound waves, as they are emitted from (or received by) these transducer array elements interfere differently for different pitch values. For a pitch above half of the wavelength that is used for ultrasound, the waves constructively interfere at angles outside oft the "main lobe", therefore causing so-called "side lobes". Thus, a pitch value of lambda/2 may be used in order to avoid side lobes, which may negatively impact image quality when beamsteering is carried out. However, a pitch value equal to lambda may also be used, if limited beam steering capabilities and some image ghosting effects are taken into account or corrected.

In this case, the reduction of complexity is proportional to the number of units components that are combined to form one transducer array of the system, for example in a range from 8 to 32.

In further possible embodiment, a beam forming architecture is implemented by a hardware beam forming module. In other words, the beam forming on a sub-array level is performed not or not only via signals from the control unit, but by a hard-wired chip design.

For example, delays are defined for inputs and/or outputs within one sub-array, such that a defined beam form is reached for ultrasound signals generated or detected by the transducer elements connected to the inputs/ouputs. In the analog domain, sensor signals for all sub-array outputs are delayed and summed into a single output channel. Signals from this common output channel may be provided to one ADC and converted to a digital signal for further data treatment, filtering, imaging and monitoring of measurements.

The number of analog-digital converters that are needed by the system is reduced from one per transducer array unit to one per sub-array, thus also reducing the amount of data that is generated. Consequently, significant benefits in power consumption and data rate may be reached.

Additionally or alternatively, a software or programming implementation of sub-array beamforming may be used in the system.

Also, the invention relates to a method for operating an ultrasound system. The ultrasound system has at least two operating modes, namely a sleep mode and a measuring mode. The control unit is configured, when the sleep mode is activated, to have a low frequency system clock running to a predetermined sleep frequency, and, when the measuring mode is activated, a high frequency system clock is set to a measuring frequency.

In particular, the method serves the purpose of operating the ultrasound system. Thus, it has the same advantages as the ultrasound system of the invention.

Further details and advantages of the present invention shall now be disclosed in the connection with the drawings.

It is shown in
- **Fig.** 1: an embodiment of the ultrasound system;
- **Fig. 2A-C**: detailed views of the transducer array of the embodiment of the ultrasound system;
- **Fig. 3A-D**: another embodiment of the ultrasound system;
- **Fig. 4**: another embodiment of the ultrasound system;
- **Fig. 5**: an embodiment of a method to operate the ultrasound system;
- **Fig. 6**: another embodiment of a method to operate the ultrasound system;
- **Fig. 7**: another embodiment of a method to operate the ultrasound system; and

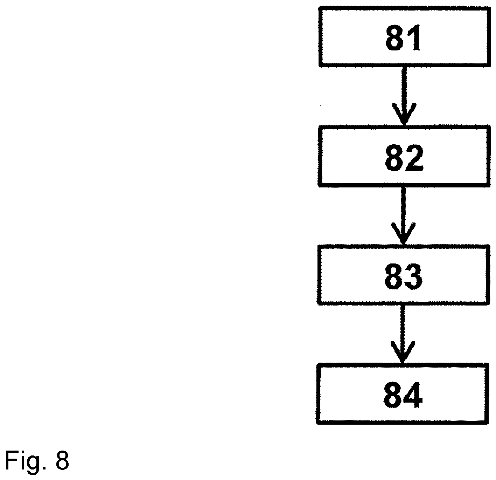

- **Fig. 8**: an embodiment of a method for correction of ultrasound systems.

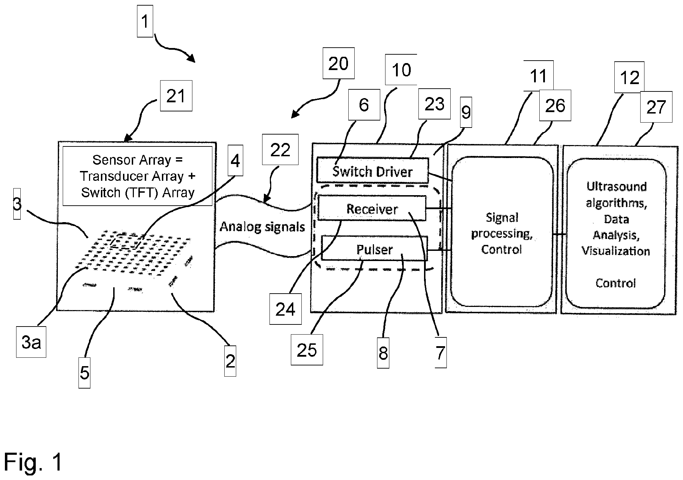

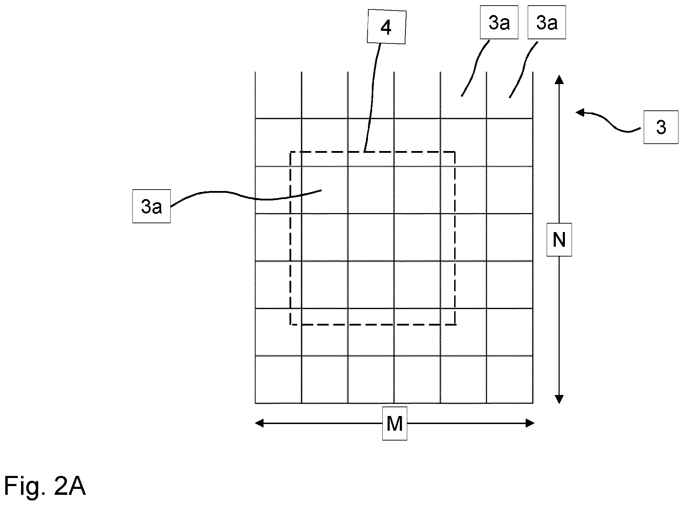

Turning to **Fig. 1** and **Fig. 2A** to **Fig. 2C****,** an embodiment of the ultrasound system is described.

**Fig.** 1 shows a schematical overview and explaining the functional blocks of the ultrasound system 1.

The ultrasound system 1 according to the embodiment is configured as a wearable ultrasound system 1, in particular for long-term monitoring of a patient's cardiac activity.

In particular, the ultrasound system can be in general an ultrasound system as disclosed by WO 2019/162485 A1. The below additional features can be further realized in this already disclosed ultrasound system of WO 2019/162485 A1.

The ultrasound sensor and support electronics can be integrated in a flexible body worn format, as it is shown in this embodiment.

In the embodiment as shown in Fig. 1, the ultrasound system is (at least partially) carried in a patch 2.

The ultrasound system 1 comprises a transducer array 3, which is configured as a piezoelectric micromachined ultrasonic transducer (pMUT) array 3 in the present embodiment.

The transducer array 3 comprises a plurality of transducer array elements 3a.

In the present embodiment pMUT elements 3a.

A subset of the transducer array elements 3a forms a sub-array 4.

In particular, each transducer array element 3a can consist of one or several sub-elements 3b, in particular pMUT sub-elements 3b or components, which are driven as a single larger transducer array "element" 3a.

In other embodiments, capacitive micro-machined ultrasonic transducer (cMUT) components can be used.

Other configurations with different numbers of transducer sub-elements 3b or a different arrangement of the sub-elements 3b are possible as well.

Furthermore, there is a switch array or switch module 5, which can more specific be embodied as a TFT array.

A further part of the ultrasound system 1 is the Analog Front-End 10 with the Switch Driver 6, the Receiver 7 and the Pulser 8.

The Switch Driver 6 can be embodied as a TFT driver module.

The Receiver 7 can be embodied as analog-digital converter (ADC).

The Pulser 8 can be a pulser module.

There can be a digital application-specific integrated circuit (ASIC) 9.

Further, there is an interface module 11 and an external unit 12/back-end unit 12.

A transducer array 21, which comprises a sensor array (e.g., like the transducer array 3 described above) and a switch/TFT array, is coupled with a unit implementing a switch driver 23, a receiver 24 and a pulser unit 25.

These units are configured to communicate via analog signals 22 with the transducer array 21.

In the embodiment, they are implemented in application-specific integrated circuits. Such ASICs can be located on the periphery of the sensor or on the Analog Front-End 10.

Also, they are used to receive and treat the signals, and communicate with a signal processing/control unit 26, which is implemented in an embedded compute system, e.g. on the Analog Front-End 10 with CPU, FPGA with memory or other units, these control and signal processing algorithms might also be implemented partly or completely in an ASIC.

Further processing of provided data is performed by a control module 27, incorporating for example ultrasound image reconstruction algorithms, data analysis, visualization and control algorithms.

In the case of a system 20 wired to an external unit 12, such as a PC, at least a part of the treatment algorithms can optionally be run on the embedded compute system and/or on the external unit 12. In a wireless case, such computational steps can be carried out in the embedded compute system or potentially at least partly in a holder.

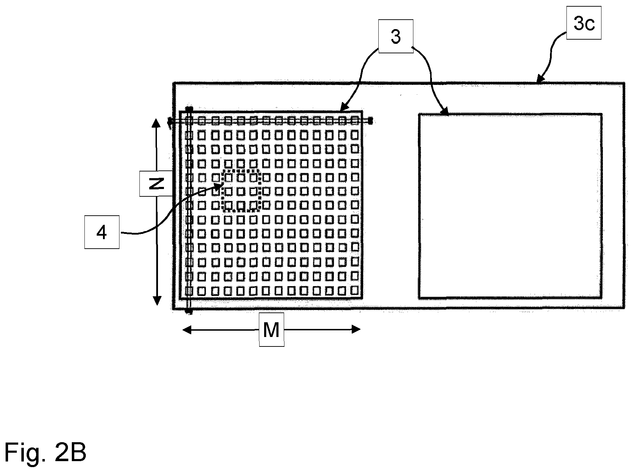

Furthermore, **Fig. 2B** shows another embodiment, wherein two or more transducer arrays 3 can form a transducer array set 3c, which is then controlled as one functional unit, i.e. "virtual" single transducer array.

In the embodiment of **Fig. 2B****,** a first and a second transducer array 3 are comprised by one transducer array set 3c. In this embodiment, the transducer array set 3c is controlled as "virtually" one single transducer array 3.

Also, **Fig. 2B** shows that the transducer array 3 comprises a plurality of transducer array elements 3a, which are arranged in a matrix with a number of N rows and M columns.

Furthermore, **Fig. 2B** shows that a subset of the transducer array elements 3a make up a sub-array 4, which is herein controlled as one unit. The sub-array 4 has a number of n rows and m columns.

**Fig. 2B** also shows that elements 3a along a row and/or a column of the transducer array 3 are interconnected with one or more interconnecting lines, a so called row/column based architecture. The elements 3a connect in parallel to the interconnects, with a switch, in particular a TFT switch, determining if an element 3a is connected or not and to which signal path it connects. Thus, rows and/or columns of the transducer array 3 can be controlled together. This allows simplifying the system by switching rows and/or columns of elements 3a together.

The transducer array 3 of the embodiment is flexible. Here, the transducer array elements 3a are arranged on a flexible substrate, e.g., comprising a flexible polymer material.

Furthermore, the ultrasound system 1 comprises a switch array which can be embodied as a thin-film transistor (TFT) array 5, comprising a plurality of TFT elements.

The switch array 5 is arranged such that its switch elements (or TFT elements when embodied as a TFT array) control corresponding pMUT elements 3a. In particular, each TFT element is assigned to one pMUT element 3a.

In the present embodiment, a TFT array 5 with 150 * 150 TFT elements is used, which are arranged in a 2D matrix with perpendicular rows and columns. The array size depends in particular on the size of the respective pMUT array 3, and it may differ in different embodiments.

Similar to the transducer array 3, the TFT array 5 of the embodiment is also flexible and/or stretchable. Here, the TFT array elements are arranged on a flexible substrate, e.g., comprising a flexible polymer material.

In particular, the TFT array 5 is arranged on the same substrate as the transducer array 3.

In particular, the integration may be monolithic or heterogeneous. In the present exemplary embodiment, the sensor layer and TFT layer may be fabricated separately and joined in a wafer-to-wafer bonding process, followed by the processing of an interconnect module.

In particular, "Heterogeneous Integration (HI)" may refer to the assembly and packaging of multiple separately manufactured components onto a single chip in order to improve functionality and enhance operating characteristics. In the present embodiment, one single array is created with a single functionality (sensing), implying a monolithic device.

In particular, attention is drawn to **Fig. 2B****,** wherein an example of a row/column architecture is demonstrated.

In the embodiment, both the pMUT array 3 and the TFT array 5 are integrated into an adhesive patch 2.

In the embodiment, the adhesive patch 2 has a size of about 10x10 cm. The size may vary in different embodiments.

The adhesive patch 2 comprises a material that is suitable for the purpose of attaching the ultrasound system 1 and in particular the transducer array 3 to a patient's skin. Therefore, biocompatible and breathable materials are used. The adhesive layer on the patch is used to attach the patch to the body and to make sure that the position of the transducer array 3 is essentially constant with respect to the patient's skin.

In another embodiment, an "acoustic matching layer" is provided in between the skin and the transducer array 3.

The system 1 also comprises a functional block for the analog control of the electronics, a so called Analog Front-End.

The Analog Front-End 10 is coupled with the transducer array 3 and the TFT array 5 for providing options such as control and read-out for the transducer array 3 and/or the TFT array 5. To this end, both data and electrical power may be transferred over the coupling between the Analog Front-End 10 and the patch 2 with transducer array 3 and TFT array 5.

According to the embodiment, the Analog Front-End can be implemented in an application specific IC (ASIC) or as circuits on the TFT layer or as discrete components or a combination thereof. All the physical components of which the analog front-end is comprised can be either integrated directly on the TFT as integrated components, on the periphery of the TFT as IC's or on a PCB (flex or rigid) physically attached to the sensor 2 through physical interconnects.

In different embodiments, the application specific IC's may be either integrated on the periphery of the TFT layer or on the PCB.

Here, a TFT driver module 6 is provided on the Analog Front End 10, which is configured to provide a low-level interface to control and manage the TFT array 5 and its individual TFT elements, respectively. In an embodiment, a gate driver is integrated with the ASIC on the periphery of the TFT layer. In another embodiment the driver circuitry could be designed partly or completely in the TFT layer.

In particular, the TFT driver module 6 of the present embodiment can be integrated together with send and receive modules for controlling the generation of ultrasound pulses and detecting ultrasound signals, respectively. All these functional modules form part of the analog front-end.

Also, a receiver 7 is provided as part of the analog-front end 10, which is configured to convert an analog input signal to a digital output signal.

Also, a pulser module 8 is provided as part of the analog front end 10, which is configured to control the transducer array 3 such that ultrasound pulses are generated. In particular, a beam-forming is performed here. Delay values may be set individually for each pulser unit of the system 1 that generates an ultrasound pulse. The delays on the different channels, in particular each pMUT element 3a, of the pulser unit will determine the focusing and the beam profile.

Also, a digital middle-end is provided , which is configured to perform part of the control operations for the ultrasound system 1, and specifically to control the activity of the transducer array 3 and the TFT array 5 for generating ultrasound pulses and detecting ultrasound signals. The digital middle-end is also configured to perform signal processing on the signals received from the analog front-end.

As part of the digital middle end, microprocessors may be provided and/or other units for performing operations for controlling the ultrasound system 1 and its electronic components.

Thus, the ultrasound system 1 of the embodiment provides an easily customized ultrasound sensor and monitoring device.

The interface module provides the possibility to build up a connection based on a wire connection and/or a wireless connection. In the present embodiment, examples for connection methods include USB and Ethernet as well as via Bluetooth and WiFi networks.

The interface module is here configured to allow for data communication and/or power connections with external units.

In the embodiment, the PCB with its components and the TFT array 5 constitute an integrated control unit and data processing unit. PCB and TFT array 5, and the transducer array 3, may in different embodiments, be configured as units that are formed separately or they may be incorporated in a common unit.

In a further embodiment, a remote or cloud based back-end unit 12 may be accessed through the interface module, either directly or indirectly via another device, such as a computer that is connected to a local or wide area network. The back-end unit may perform operations such as to reading and storing data, data analysis, configuration of the device, providing further information and administrating access the acquired data based on an authentication system.

Turning to **Fig. 2A****,** a detailed view of the transducer array 3 of the embodiment of the ultrasound system 1 is described.

The transducer array 3 has a matrix of pMUT elements 3a, which are only schematically shown in a small number.

The system 1 is configured such that a sub-array 4 of pMUT elements 3a may be defined. The elements 3a of a sub-array 4 are treated as one functional unit for generating ultrasound pulses and/or detecting ultrasound signals.

In the embodiment, an ASIC performs steps for defining sub-arrays 4 within the transducer array 3. The definition of sub-arrays 4 may be carried out in different ways, e.g., by way of the chip design or by other units controlling the transducer array 3.

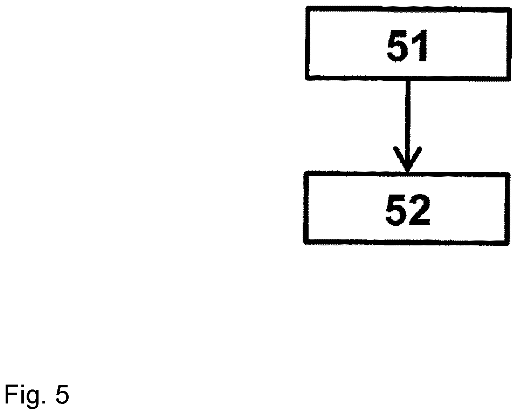

In the present embodiment, the transducer array 3 has N=150 rows and M=150 columns, which are ordered in a two-dimensional matrix. On the other hand, a sub-array size value of 16 rows by 16 columns is defined. Thus, groups of 162 elements 3a are grouped into one sub-array 4. This leads to a considerable reduction in system complexity and data volume, respectively. This aspect is further described below with reference to **Fig. 5****.**

Herein, the interrelated elements 3a are chose such that they are neighboring to each other.

In further embodiments, this definition of sub-arrays 4 may be configured in different ways, such as, in a configuration step, by inputting a predetermined sub-array size value, which defines the number of pMUT elements 3a to be included in a sub-array 4, and/or by inputting a location of a sub-array 4 within the pMUT array 3. Also, the form of a sub-array 4, i.e., its dimensions size, may be determined by an input value.

In one embodiment, the sub-array size may be defined during the system development. In the products itself, the sub-array size can be fixed, without an option to dynamically reconfigure this value; in this case, a fixed size would be used.

Said values to control how sub-arrays 4 are defined may also be determined by the digital Middle End and/or digital back-end, part of this functionality might be implemented in an ASIC or another control element of the ultrasound system 1, or by an input over the interface module, in particular depending on an algorithm. Such an algorithm for determining parameters for defining the sub-arrays 4 may use input values such as data/image quality requirements, a signal-to-noise ratio, information about factors interfering with the operation of the system, user input and configuration information, and/or other parameters.

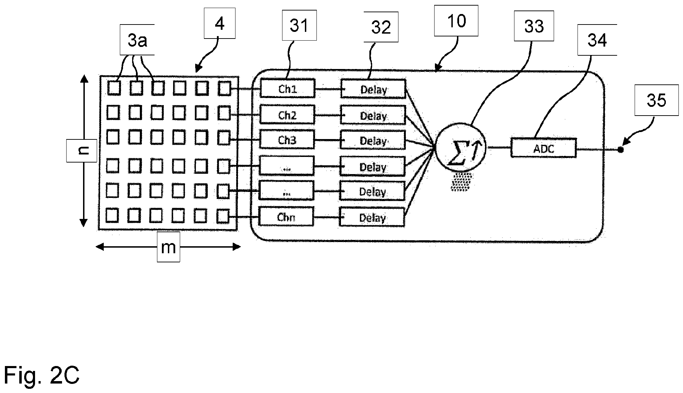

Turning to Fig. 2C, an embodiment for implementing a row/column based micro beam-former is schematically described.

A sub-array 4 has a number of n row and m columns, and is connected to a micro beam-former 30. In this specific case, the n rows of transducer array elements 3a are combined into one output channel 31 each. These output channels are named "Ch1" to "Chn".

For the transducer array element 3a of the sub-array 4, the micro beam-former 30 combines the n analog input channels 31 from the sub-array 4 into one single digital output channel 35.

To this end, a delay operation is performed by delay units 32 and a sum unit 32 is subsequently used to coherently add the n analog inputs together.

An analog-digital converter 34 is used to convert the resulting analog signal to a digital signal, which is output through a digital output channel 35.

In further embodiments, each channel 31 can do some analog processing of the input signals before its signal is combined in the analog beam-former 30.

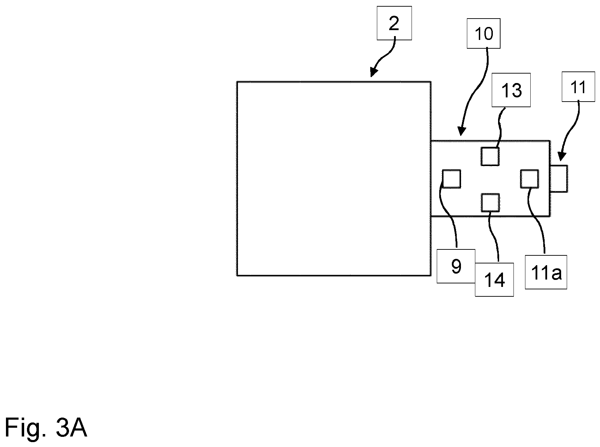

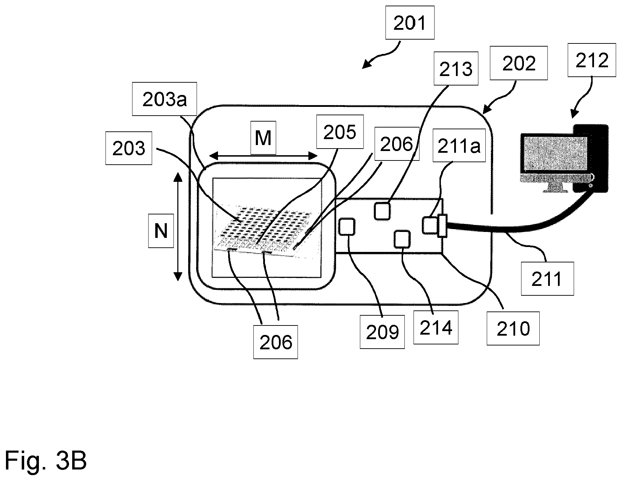

Turning to **Fig. 3A** to **Fig. 3E**, other embodiments of the ultrasound system are described. Reference is made to the embodiments of the ultrasound system as described above. Figures 1 and 3A to 3D are meant to present analog embodiments of the system, although different aspects are highlighted and different elements are explicitly shown.

In **Fig. 3A****,** a PCB 10 is shown connected to the patch 2. The transducer array 3 and the TFT array 5 are not shown, since they are also integrated with the patch 2. Also, the interface module 11 is directly connected to the PCB 10.

On the PCB 10, circuits for controlling the system 1 are shown.

The ASIC 9 is provided on the PCB 10.

Also, an interface control module 11a is provided and implemented with another IC. It provides a low-level interface to the interface module 11, thereby allowing to control the interface module 11.

Also, a memory module 13 is provided, herein specifically configured as a random access memory (RAM) module 13.

Also, a power management module 14 is provided.

In another embodiment, a power source is provided, for example a battery as further explained below with reference to **Fig. 3E**. In this case, access to the stored power is provided to the system components via the power management module 14. In this case, the interface module 11 may be configured to provide a wireless data connection, in particular to a control unit.

In the present embodiment, another way of providing power to the system may be used, such as a connection to an external power source or an external power grid.

The ASIC 9, interface module ASIC 11a, the memory module 13 and the power management module 14 may be implemented in different ways, some of which may be known in the art. For example, a microprocessor, an ASIC chip and/or other units may be used.

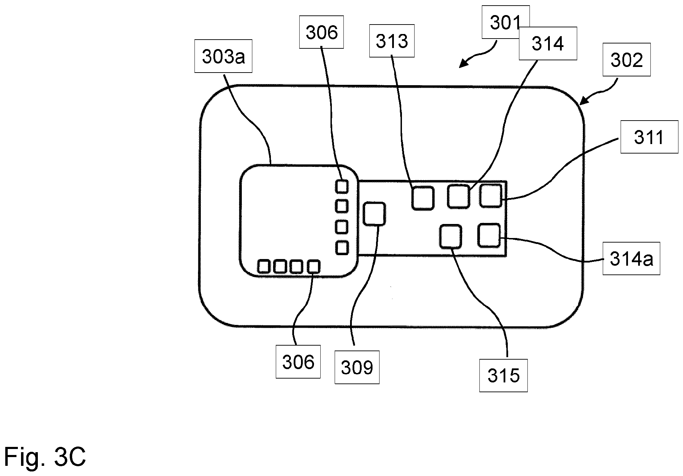

Turning to **Fig. 3C****,** another view of an embodiment equivalent to the one of Fig. 3A is described.

In this embodiment, a system 201 comprises a unit 203a, in which a transducer array 203 and a TFT array 205 are integrated. A flexible PCB 210 is coupled to this unit 203a, and both are integrated in a patch case 202.

The PCB 210 might be rigid, flexible, stretchable or a hybrid combination thereof.

The system 201 contains a module 206 with an ASIC with 16 channels, containing an analog front-end functional block and optionally a complete or partial digital middle/back end. The ASIC module 206 can be integrated either on the sensor unit 203a or on the PCB 210.

In particular, the flexible PCB 210 is configured such that it is flexible on the top of the sensor, in particular on top of the sensor unit 203a. The physical link between the PCB 210 and the sensor unit 203a being made by interconnects such as in particular flexible interconnects.

The flexible PCB 210 comprises components for an embedded computer system, such as a control device 209, comprising for example CPU, FPGA and/or other elements.

The flexible PCB 210 further comprises a physical interface module such as e.g. a USB connector 211 and a USB transceiver 211a, by which the system 201 is coupled to a PC 212.

The flexible PCB 210 also comprises a RAM module 213 and a power management IC 214.

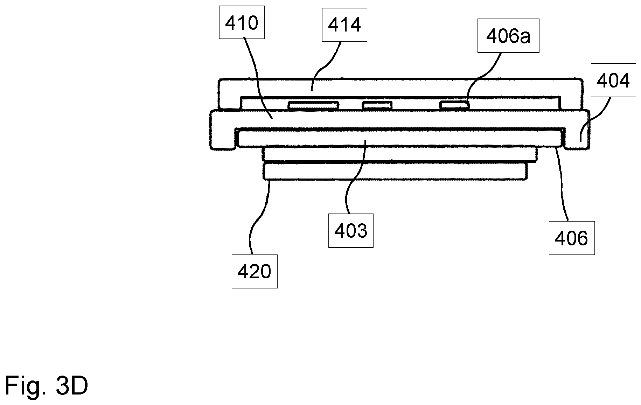

Turning to **Fig. 3D****,** another view of an embodiment equivalent to the one of **Fig. 3A** is described.

A system 301 comprises a flexible packaging, in particular a patch case 302, into which a sensor unit 303a and a flexible PCB 310 are integrated. The flexible PCB 310 is coupled to the sensor unit 303a.

The PCB 310 might be rigid, flexible, stretchable or a hybrid combination thereof.

The system 301 contains a module 306 with an ASIC with 16 inputs, containing an analog front-end functional block and optionally a complete or partial digital middle/back end. The ASIC module 306 can be integrated either on the sensor unit 303a or on the PCB 310.

The flexible PCB 310 has a control device 309, a wireless transceiver 311, a memory module 313, a battery gauge 314, a wireless/wired charger 314a and a power IC 315 integrated on it.

In particular, the flexible PCB 310 is configured such that it is flexible on the top of the sensor, in particular on top of the sensor unit 303a. The physical link between the PCB 310 and the sensor unit 303a being made by interconnects such as in particular flexible interconnects.

Also, a flexible battery may be provided.

Turning to **Fig. 3E,** a schematic cross-section of an embodiment, such as the embodiment of **Fig. 3D****,** is described.

The system 401 comprises a sensor 403.

An acoustic matching and/or glue layer 420 is applied on one side onto the sensor 403, to enable good contact to a subject's skin.

On the other side of the sensor 403, a periphery 406 section is arranged. In particular, electronics for controlling the sensor 403 are arranged in the periphery 406.

As a layer above the periphery 406, interconnects 404 are arranged. In particular, the interconnects 404 can connect elements of the sensor 403, e.g., in a row/column architecture.

In a layer above the interconnects 404, IC elements 406a are arranged.

On top of the above-described arrangement, a flexible battery 414 is arranged. This flexible battery 414 might also be arranged next to IC elements 406a and/or the PCB 410.

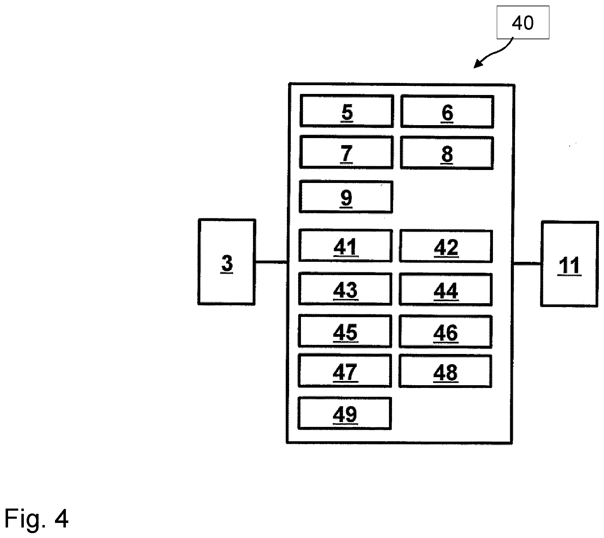

Turning to **Fig. 4****,** a schematic view of another embodiment is shown. The shown components may also be present in the embodiments described above; similar or equivalent elements have the same reference numerals and are not described again in detail.

Some or all of the elements and modules shown in **Fig. 4** may be implemented in different units, such as separately formed devices, or they may be implemented by way of program modules within the same computational unit and/or a digital memory device.

The combination of elements and modules of this embodiment is to be understood as exemplary, and as comprising a large number of optional features, which are not necessarily linked to each other or to be combined in one embodiment of the invention.

In **Fig. 4****,** the transducer array 3 is shown, which is linked to a control unit 40 (controlling the transducer array 3), which may be configured at least partially on a PCB 10.

The connection between the transducer array 3 and the control unit 40 is a direct and cable-bound connection. However, in an alternative embodiment, an indirect and/or wireless connection is possible.

For instance, the control unit 40 can be attached to the patch 2 (possible for wireless patches and wired patches). Further, there can be an additional control unit in a separate computer, in particular for the wired version.

The transducer array 3, configured for providing ultrasound waves and/or receiving ultrasound signals, comprises a plurality of transducer array elements 3a.

The control unit 40 comprises the TFT array 5, which was already described above.

The control unit 40 further comprises a dedicated data processing unit 41, which may in different embodiments comprise some of the other modules (as specified below with reference numerals 41 to 49) or which may in different embodiments be implemented by several individual modules. The data processing unit 41 receives and processes data from the transducer array 3.

Moreover, the control unit 40 comprises a reference module 45.

Also, the control unit 40 comprises a volume reconstruction module 46, in particular for reconstructing the volume of a target organ or part of a target organ which is in this embodiment the left ventricle of a subject equipped with the ultrasound system 1. Furthermore, the control unit 40 comprises a selected monitoring unit 47, in particular for monitoring the left ventricle.

Further, the control unit 40 comprises a correction module 44.

Also, the control unit 40 comprises a complexity reduction module 42.

Additionally, the control unit 40 comprises a deformation correction module 43.

In addition to that, the control unit 40 comprises an artifact determination module 48.

Furthermore, the control unit 40 comprises a phase determination module 49.

The system 1 according to the embodiment is controlled by integrated support electronics. The support electronics comprise mixed signal ASIC elements distributed along the periphery of the sensor. The sensor may comprise the transducer array 3. The ASIC elements could also be located on the PCB 10.

Also, the rest of the electronics is integrated on the flexible PCB 10, which is in turn connected to the sensor.

The ASIC elements are distributed along the edge of the flexible ultrasound sensor and have some or all of the following functions:
- Actuating the mechanical transducers, i.e., the transducer array elements 3a;
- Processing and digitizing signals received by the transducer array elements 3a;
- Controlling the switching of the TFT array 5; and/or
- Dynamically reconnecting the analog channels, i.e., from different transducer array elements 3a, to different sets of columns/rows.

The flexible PCB 10 contains a digital "master" ASIC 9 and/or a microprocessor, for performing digital signal processing, and control of the other ASIC elements. The digital signal processing and/or control may also be implemented in part or completely on the mixed signal ASICs distributed along the periphery and/or edge of the sensor (e.g. routed to the backside of the sensor on a PCB by means of interconnects).

By performing row/column addressing of the pMUT elements 3a of the transducer array 3, the complexity of the system 1 is reduced from N*M to N+M, wherein N and M represents the amount of rows and columns in the array. In case of a fully wired array

(i.e., when each transducer array element 3a is controlled individually) each element 3a can be addressed individually (hence the N*M complexity), while for a row/column based architecture the elements 3a along the same row or column are connected together and addressed as a single unit, reducing the complexity to N+M. Each unit could still consist of multiple interconnect lines forming the connections between the external nodes and the internal array elements. Each interconnect line can carry a different signal, e.g. to provide a signal line, a ground line or to provide a control signal for switching the TFT transistors.

A hardware beam-former may be used to combine, e.g., 16 analog channels, which connects each to an interconnected unit of transducer array elements 3a, to a single ADC 7, i.e., one digital channel. The number of combined elements 3a determines the factor by which the system complexity is reduced. In particular, a plurality of ADCs 7 may be provided and these may be linked to a defined sub-array 4 of transducer array elements 3a.

In the embodiment, a sub-array 4 is used as one functional unit, e.g., a "single entity" for scanning the full array (a unit cell). This single entity is electronically controlled and/or scanned over the larger transducer array 3. In particular, the sub-arrays 4 are electronically "shifted" per single row/column of the transducer array 3.

In the present embodiment, a sub-array 4 is electronically controlled as a single unit. In particular, there is one ASIC for each sub-array 4.

In further embodiments, multiple sub-arrays 4 may be provided for each ASIC. The number is determined by the accessible power, cost restrictions, acceptable compactness and/or speed requirements for the system.

By using several ASICs for controlling individual sub-arrays or controlling multiple sub-arrays per ASIC, faster scanning is possible, since several measurements, in particular "single entities" or "functional units", are operated in parallel.

In the following, aspects and methods of operating an embodiment as described above are described. In particular, the following description is based on an embodiment similar to the one of **Fig. 4****.**

When the transducer array 3 is placed on the body (thorax) of the subject, it does not have any prior information on the features of the body on which it is placed. Thus, the reference module 45 provides a detailed scanning of the region of the total transducer array 3 (matrix of transducers). This scanning will allow to detect the left ventricle as feature of interest/target. Any method known in the art can be used for feature detection, e.g., correlation with known feature, neural networks etc. Furthermore, this scanning allows to detect incorrect placement of the transducer array 3 on the thorax of the subject, and/or it may be evaluated, whether the left ventricle is only partially visible from the position of the transducer array 3.

Based on the reference ultrasound data obtained by the reference module 45, the volume reconstruction module 46 reconstructs the volume of the left ventricle by providing a collection of collimated ultrasound beams by a sub-array 3a of the transducer array 3.

Thus, the volume reconstruction module 46 can control a location of a sub-array 4 within the plurality of transducer array elements 3a. Also, the volume reconstruction module 46 can control, for each sub-array 4, a focus depth and/or a steering angle for an ultrasound sending beam profile and/or an ultrasound receiving beam profile implemented by the sub-array 4 of transducer array elements 3a; such a beam-forming algorithm may be implemented as a software module for a programmable unit and/or by chip design, e.g., in an ASIC.

Another method could use different sparse pulse-echo measurements of the left ventricle, and use these measurements to determine the deformation against a more detailed reference volume, which would have been collected in an earlier measurement, i.e., it would track the deformation of the reference volume over time using only sparse measurements. Yet another method could use the imaging data directly to track the deformation directly against a reference image.

The artifact determination module 48 can be used for identification of artifacts and avoiding obstructions to ultrasound imaging caused by artifacts, such as air bubbles between the transducer array and the skin of the patient equipped with the system or bones, e.g. ribs. In particular, strong ultrasound reflection of the ribs may be used for identification. The artifact determination module 46 in general may combine groups of transducer array elements 3a into a sub-array 4.

Combining of groups of transducer elements 3a into a sub-array 4 can alternatively be done by the complexity reduction module 42.

Also, the complexity reduction module 42 may be implemented as a software module, comprising instructions for computational operations that implement the function of complexity reduction. Such a software module may in particular be implemented together with the control unit 40.

Specifically, the complexity reduction module 42 may be comprised, at least partially, by the control unit 40 and/or by the data processing unit 41.

Further, the selected monitoring unit 47 is configured to monitor the left ventricle (by means of the sub-array 4).

In particular, the computational load may be further reduced by only performing a detailed scanning of the volume at discrete time instances of the cardiac cycle:
The phase determination module 49 is configured for determining cardiac cycle phases in particular end-systolic phase and end-diastolic phase, as for evaluation of the stroke volume only the end-diastolic and end-systolic phases are of importance. For this a single (alternatively a few) ultrasound pulse-echo measurement(s) is/are done through a carefully chosen position of the left ventricle or other reference, from which the cardiac cycle can be determined. This is done several times through the cardiac cycle so that a good graph can be obtained of the contraction over time, e.g., 40 to 200 "samples" could be taken over the full cardiac cycle. From these sampled curves, the end-diastolic and end-systolic phases are detected by analyzing the derivative to determine min/max in the curve(s). Alternatively, several cycles can be measured, which allows for accurate estimation of the end-systolic/diastolic phases. The regularity of the heart rhythm then allows to determine the next instance of the end-diastolic/end-systolic phases. Another option may be to train a neural network, which takes as input the time series and gives as an output the end-diastolic and systolic phases. Yet another option would be to identify focus on a feature of the heart which provides a good indication of the heart cycle, such as e.g. a valve of a heart chamber. The phase can also be determined by monitoring the heart cycle with other signals like e.g. an ECG signal.

Therefore, a detailed volume scanning at the end-diastolic and end-systolic phase is enabled by means of the selected monitoring unit 47.

For instance, once the end-diastolic and systolic phases have been detected by the phase determination module 49, the system 1 can switch from the phase determination module 49, and send a single "scanning" beam to the volume reconstruction module 46 and send a sufficient amount of beams to reconstruct the volume.

For data treatment, in particular for monitoring and/or clinical applications, a cardiac output variation over certain time periods, e.g., over a day, may be determined by the system 1. Also, motion data may be provided and, e.g., linked to measured cardiac activity, such as heart rate and other parameters. For example, an accelerometer may be integrated or connected to the system 1. Also, a movement of the patient's thorax may be determined with an accelerometer. Furthermore, the influence of breathing and physical activity may be considered. A movement of the patient may be measured, e.g., a rhythm of breathing. Furthermore, the lungs may be monitored by the ultrasound system as well.

Also, changing properties of the transducer array 3, e.g., a pMUT array 3, may be analyzed in order to obtain information on the deformation of the transducer array 3.

Also, an automatic evaluation may be carried out to determine whether the signal-to-noise ratio is too low, i.e., below a predetermined limit, and the reliability of measurements is affected.

In particular, ultrasound based pulse-echo measurements using focused beams may be used to sample a probe volume.

As a way to reduce the volume of acquired data, which needs to be processed and analyzed during monitoring of cardiac activity, sampling may be limited to end-diastolic and/or end-systolic volumes of a heart chamber of or another volume.

In order to track a heart rhythm, a single reference beam may be used to track heart motion and evaluate end-diastolic and/or systolic phases.

To make sure that the monitoring provides most reliable data, different measures may be taken: Ultrasound pulse-echo measurements may be used together with a sampling of selected, reduced measurements of few spatial data points. Also, ultrasound pulse-echo measurements may be used together with a varying pulse repetition frequency, wherein the pulse repetition frequency is reduced outside of defined measurement windows. The pulse repetition frequency may relate to a regular interval; also, pulse-echo measurements may be limited to well-defined time windows. For example, a higher pulse repetition frequency may be set within these time windows, in order to gather more pulse-echo measurements and perform a denser sampling of the volume on interest, while the pulse repetition frequency is set lower outside the time windows, e.g., to track the phase of the heart contraction with lower time resolution.

The monitoring may comprise, in particular with a given frequency, steps of calculating a volume, calculating the volume of ellipse in order to approximate a volume and/or volume change, segmentation and integration of image data, detecting a base line value, calibrating the system and/or monitoring strain, in particular on the transducer array, in order to estimate deformation and/or a dynamic form factor of the transducer array.

In particular, monitoring and calibration are implemented as two distinct steps in the operation of the system, in particular where the transducer array is included in a flexible patch:
a) The patch is applied to a patient's body.
b) The patch would perform a calibration step (detecting its deformed shape due to the shape of the thorax).
c) The system would perform monitoring and tracking the volume of the heart over time.

During monitoring, potentially one would need to correct for dynamic shape changes of the patch, e.g., due to breathing or movement of the patient. This could be done by, e.g., a) performing a recalibration at regular intervals, b) measuring deformation with strain monitoring, c) using a tracking algorithm for the shape deformation.

The monitoring of the volume could be done in different manners:
a) Establish a detailed baseline shape of the ventricle, and track the changes in this baseline shape over time with limited number of ultrasound based pulse-echo measurements.
b) Measure with a limited number of ultrasound based pulse-echo measurements and establish directly the volume from these.

In particular, image based metrics may be used for the calibration step. This may enable a deformation-free reconstruction of images even from a deformed transducer array, i.e., without reconstruction of the concrete form factor of the transducer array. E.g., wrong shapes may be assumed and true images may be reconstructed based on these wrong shapes.

Also, shape estimation for the transducer array may be performed based on deformation-free reconstruction data.

Measurements may be carried out by generating reference beams towards a diffuse reflector, and detecting the reflected ultrasound signals with another known sub-array 4, in order to evaluate displacement of the sub-arrays 4 relatively to each other, which would allow for estimating the deformed shape of the array 3.

Also, changing properties of the transducer array 3, e.g., a pMUT array 3, may be analyzed in order to obtain information on the deformation of the transducer array 3.

Different detection methods may be used, in particular for monitoring cardiac activity in a patient: A left ventricle may be detected and targeted for measuring, in particular measuring its volume and the dynamic change in volume.

Moreover, ribs and other obstructions in the patient's body may be detected in order to reduce noise in the measurements and to optimize the choice of a suitable ultrasound beam-forming and focusing. In particular, strong ultrasound reflection of the ribs may be used for identification.

In order to define a sub-array 4 of the transducer array 3, several steps may be provided and carried out, individually or in combination with each other. A positioning step may be carried out for sub-arrays 4 in order to control their position within the transducer array 3, i.e., for choosing suitable elements 3a and assigning them to the sub-array 4. Also, a sub-array's position may be shifted, in particular randomly, to avoid long term stress on specific elements 3a, leading to degradation of the elements, in particular pMUT elements 3a.

The use of ASIC elements may improve the security of the device, since the respective control methods are not easily manipulated, at least not without direct physical access to the device.

In one embodiment, the ultrasound system 1, in particular for cardiac monitoring, comprises a transducer array 3 with a plurality of transducer array elements 3a, a control unit 40, which is coupled to the transducer array 3, for controlling the transducer array 3, and a data processing unit 41 for receiving data from the transducer array 3 and processing the received data. Therein, the ultrasound system 1 further comprises a complexity reduction module 42, which is configured to define at least one sub-array 4 of transducer array elements 3a and to control the at least one sub-array 4 as a functional unit for sending at least one ultrasound pulse and/or receiving at least one ultrasound signal.

In one embodiment, the ultrasound system 1, in particular for cardiac monitoring, comprises a transducer array 3 with a plurality of transducer array elements 3a for providing ultrasound waves, a control unit 40, which is coupled to the transducer array 3, for controlling the transducer array 3, and a data processing unit 41 for receiving data from the transducer array 3 and processing the received data. Therein, the system further comprises at least one reference module 45 configured for obtaining reference ultrasound data, at least one volume reconstruction module 46 configured for reconstructing the volume of a target organ or part of a target organ, and at least one selected monitoring unit 47 configured for monitoring the volume of the target organ and/or part of the target organ.

In one embodiment, the ultrasound system 1, in particular for cardiac monitoring, comprises a transducer array 3 with a plurality of transducer array elements 3a, a control unit 40, which is coupled to the transducer array 3, for controlling the transducer array 3, and a data processing unit 41 for receiving data from the transducer array 3 and processing the received data. Therein, the ultrasound system 1 has at least two operating modes, namely a sleep mode and a measuring mode. The control unit 40 is configured, when the sleep mode is activated, to have a low frequency system clock running at a predetermined sleep frequency, and, when the measuring mode is activated, to set a high frequency system clock to a measuring frequency.

In one embodiment, the ultrasound system 1, in particular for cardiac monitoring, comprises a transducer array 3 with a plurality of transducer array elements 3a, wherein the transducer array 3 is flexible, a control unit 40, which is coupled to the transducer array 3, for controlling the transducer array 3, and a data processing unit 41 for receiving data from the transducer array 3 and processing the received data. Therein, the system 1 further comprises at least one correction module 44 configured for correction of effects in analog ultrasound signals caused by bending of the flexible transducer array 3.

In one embodiment, the ultrasound system 1, in particular for cardiac monitoring, comprises a transducer array 3 with a plurality of transducer array elements 3a, a control unit 40, which is coupled to the transducer array 3, for controlling the transducer array 3, and a data processing unit 41 for receiving data from the transducer array 3 and processing the received data. Therein, the ultrasound system 1 further comprises a deformation correction module 43, which is configured to perform a deformation correction step, wherein deformation information is determined for the transducer array 3.

The embodiments may be freely combined with each other. Further modules as described above, for example with reference to figure 4, may be provided optionally to allow further functionalities as described in this specification.

Turning to **Fig. 5****,** a method for operating the ultrasound system 1 is described. The following description is based on the embodiment of the ultrasound system 1 described above with reference to, e.g., **Fig. 4****,** but other embodiments of the ultrasound system 1 may be suited as well.

In this context, "complexity reduction" denotes a reduction of the amount of read and/or write channels needed to address the sensors, in particular the transducer array elements 3a or a sub-array 4.

This can be reached by
a) combining sub-elements 3b into a single functional element 3a (cf. **Fig. 2A****);**
b) connecting elements 3a together in a row/column architecture, leading to a complexity reduction from N*M to (N+M) (cf. **Fig. 2B****);**
c) driving a sub-array 4 instead of the full transducer array 3 at once, leading to a complexity reduction from (N+M) to (n+m); and/or
d) using a micro beam-former to combine the n (or m) analog input channels 31 of a sub-array 4 into a single digital input channel 35 (cf. **Fig. 2C****).**

In a step 51, a plurality of sub-arrays 4 is defined within the transducer array 3. In a subsequent step 52, the individual sub-arrays 4 are controlled as functional units for sending ultrasound pulses and/or receiving ultrasound signals.

In the system of the embodiment, the steps 51, 52 are performed by the complexity reduction module 42, which is comprised by the control unit 40 of the ultrasound system 1. Herein, the complexity reduction module 42 is configured to control other units and modules, especially the TFT driver module 6, the receiver module 7, and/or the pulser module 8, depending on whether a send or a receive cycle is active.

The steps 51, 52 may be performed in a cyclic manner, i.e., they may be repeated for example at given intervals or triggered by predefined events and/or control signals.

In one embodiment, the system is configured such that the sub-array 4 is defined dynamically and may change its position.

In the present embodiment, the sub-arrays 4 are defined such that there is no overlap between the transducer array elements 3a of separate sub-arrays 4, i.e., no transducer array elements 3a are "shared" among different sub-arrays 4.

In another embodiment, a transducers array set 3c comprises at least two transducer arrays 3 and the control unit 40 is configured to control the transducer arrays 3 as one functional unit.

Also, in an embodiment, the transducer array 3 may be controlled according to a row/column based architecture.

Furthermore, a sub-array size may be predetermined or provided or determined by an algorithm, and sub-arrays 4 of the sub-array size are defined based on this value. The transducer array 3 is then operated based on these individual sub-arrays 4.

Also, the transducer elements 3a are controlled by the control unit 40, in particular via the TFT array 5. The transducer array elements 3a of each sub-array 4 detect an analog ultrasound sensor signal, and produce an analog signal. This analog signal is provided to the receiver module 7.

In particular, analog signals from the transducer array elements 3b of one sub-array 4 are processed by one common receiver module of the system 1. In further embodiments, different receiver modules 7 may be provided, e.g., in order to speed up data acquisition and processing.

Furthermore, a micro beam-forming architecture can be implemented by a hardware beam-forming module 43, such as a specialized ASIC element and/or another element of the control unit of the system 1.

Also, data may be filtered after it has been received from the transducer array 3. This may be carried out by a filter module 44, which may act on analog and/or digital signals. Depending on which filter is applied, filtered data is obtained with a reduced spatial and/or temporal resolution.

In another embodiment, a filter can be configured by adjusting filter properties. Thus, filters may be activated and deactivated, the impact of a filter on the data may be adjusted and/or a resolution of the filtered data may be adapted, e.g., to the needs for monitoring specific organs and functions, imaging purposes, or device-specific properties.

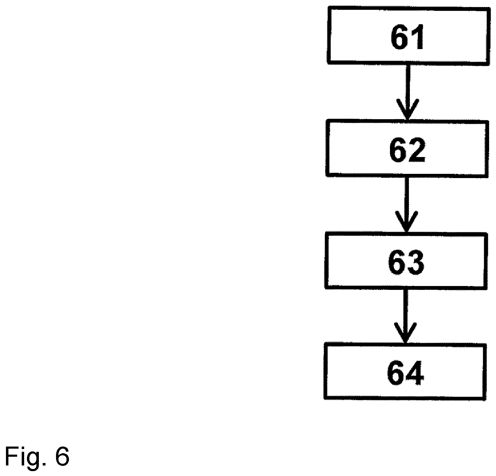

Turning to **Fig. 6****,** another embodiment of a method for operating the ultrasound system is described. The following description is based on the embodiment of the ultrasound system described above with reference to, e.g., **Fig. 4****,** but other embodiments of the ultrasound system 1 may be suited as well.

In a first step 61 of the method, a measuring mode of the ultrasound system is active. Thus, the system is configured to perform measurements using the transducer array 3.

In particular, the measurements are controlled by a system clock, which is comprised by the control unit 40. This system clock provides a frequency at which measurement steps such as generating ultrasound pulses and receiving ultrasound signals, as well as processing and analyzing of collected data are carried out.

In a step 62, the control unit 40 receives a sleep signal and subsequently activates a sleep mode of the system. According to the embodiment, the sleep signal is generated after a predetermined time duration, wherein no measurements were carried out by the system and/or wherein no data queries were received. In another embodiment, the sleep signal is received from an external unit via the interface module 11.

In response to the sleep signal, a sleep-on sequence is performed, and during this sequence, the control unit 40 shuts down a series of modules, except those that are necessary to power up the system in a later step. In particular, a wake-up controller module, an interrupt controller module, and/or a timer module are kept running during the sleep mode.

With the sleep mode activated, the system clock is configured to set a standby frequency to a value between 25 and 50 kHz, preferably to a value of 32 kHz.

In another step 63, a wake-up signal is received and the control unit 40 is configured to activate the measuring mode of the system. To this end, a sleep-off sequence is performed, wherein modules for performing measurements and providing data are subsequently started. In particular, the sleep-off sequence may be configured such that those modules, which were deactivated during the sleep-on sequence, are activated again.

According to the embodiment, the first module to be activated comprises a phase-locked loop (PLL), which is configured to provide a measurement frequency signal to the system clock. Thus, the system will advantageously be provided with a high frequency clock after performing the sleep-off sequence.

With the measuring mode activated, the system clock is configured to set a measurement frequency to a value of about 200 MHz, preferably between 150 and 250 MHz.

In a further step 64, the system 1 is configured to perform periodic measurements for monitoring the cardiac activity of a patient. These measurements are timed by the system clock, which is at a higher frequency value and allows high-frequency measurements.

In one embodiment, a common system clock is used to provide the low or high frequency in sleep and measuring mode, respectively. Thus, when the measuring mode is activated, the system clock is set to the measuring frequency, and when the sleep mode is activated, the system clock is set to the sleep frequency.

In another embodiment, a low frequency system clock and a high frequency system clock are provided. Therein, the low frequency system clock has the sleep frequency, while the high frequency system clock is set to the higher measuring frequency, once the measuring mode is activated.

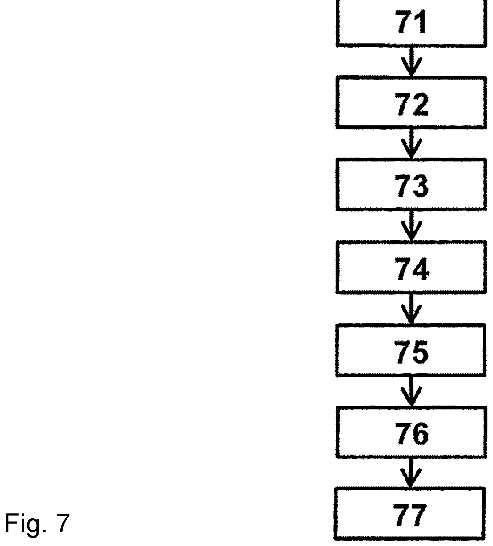

Turning to **Fig. 7****,** another embodiment of a method for operating the above-described ultrasound system 1 is described. The following description is at least in principal based on the embodiment of the ultrasound system 1 described above with reference to, e.g., **Fig. 4****,** but other embodiments of the ultrasound system 1 may be suited as well.

The method comprises the steps 71-77.

In the system 1 of the embodiment, steps 72 and 73 are obtained by the reference module 45, step 74 is obtained by the artifact determination module 48, step 75 is obtained by the phase determination module 49, step 76 is obtained by the volume reconstruction module 46 and step 77 is obtained by the selected monitoring unit 47.

The respective modules/units are comprised by the control unit 40 of the ultrasound system 1.

The steps 72, 73, 74, 75 76 and/ or 77 may be performed synchronously. Further, steps 72-77 can be performed in a different order as mentioned below.

The steps 72-77 may be performed in a cyclic manner, i.e. they may be repeated for example at given intervals or triggered by predefined events and/or control signals.

In a step 71, a transducer array 3 with a plurality of transducer array elements 3a for providing ultrasound waves is placed on the body of a subject.

In a step 72, a reference ultrasound image is obtained by scanning.

In a step 73, a target organ and/or part of a target organ is detected (in this embodiment, the left ventricle).

In this embodiment, step 72 comprises two different phases of scanning which differ in terms of resolution. In particular, step 72 is characterized by starting with a faster scanning (low-resolution scanning), which is sufficient for detecting the general region of interest. A more detailed scanning of this region of interest follows, with a higher resolution, to more accurately detect e.g. features, artifacts and/or regions which need to be monitored (i.e. the region of the left ventricle). In general, there may be more than two different phases of scanning differing in terms of resolution. Also, a constant increase of resolution may be generally possible.

Based on the results of step 72, the left ventricle is detected in step 73.

In a step 74, possible artifacts such as rips and/or air bubbles between the transducer array 3 and the skin of the subject are identified. Obstructions to ultrasound signals caused by such artifacts may be avoided by reconfiguration of a sub-array 4, cf. **Fig. 4****.**

In a step 75 cardiac cycle phases are determined, in particular the end-systolic and the end-diastolic phase.

In a step 76, the volume of the left ventricle is reconstructed.

In the embodiment, step 76 comprises scanning at least partially the volume of the left ventricle without producing an image.

The scanning may be based on ultrasound based pulsed echo measurements with focused beams, wherein ultrasound beams are beam-formed by selecting a subset of transducer array elements 3a of the transducer array 3 as a sub-array 4 within the transducer array elements 3a and applying different phases to individual and/or sets of transducer array elements 3a. Data related to a single pulse-echo measurement provides the information of two boundary points of the volume of the left ventricle, wherein the volume can be reconstructed from a point cloud based on interpolation between different points of the point cloud.

Also, the volume may be reconstructed using the sparse point cloud and determining the deformation against a more detailed reference volume which would have been collected in an earlier measurement, i.e., tracking the deformation of the reference volume over time with sparse measurements. Yet another method could use the imaging data directly to track the deformation directly against a reference image.

In a step 77, the volume of the left ventricle is monitored, only at the end-systolic and the end-diastolic phase.

Note that the target organ (here the left ventricle) may in general comprise a heart or blood vessel and the part of the target organ may comprise a ventricle or atrium. In addition to targeting an organ other volumetric features inside the body may be imaged, such as e.g. the volume of a blood inside the body due to e.g. internal hemorrhaging.

## Claims

1. Ultrasound system (1), in particular for cardiac monitoring, comprising
- a transducer array (3) with a plurality of transducer array elements (3a);
- a control unit (40), which is coupled to the transducer array (3), for controlling the transducer array (3); and
- a data processing unit (41) for receiving data from the transducer array (3) and processing the received data;
wherein the ultrasound system (1) has at least two operating modes, namely a sleep mode and a measuring mode; and
wherein the control unit (40) is configured, when the sleep mode is activated, to have a low frequency system clock running at a predetermined sleep frequency, and, when the measuring mode is activated, to set a high frequency system clock to a measuring frequency.

2. Ultrasound system (1) according to claim 1,
**characterized in that**
the transducer array (3) is flexible and/or stretchable.

3. Ultrasound system (1) according to one of the preceding claims, **characterized in that**
the transducer array (3) is integrated into an patch (2) for fixing the transducer array (3) to a patient's body by means of an adhesive and/or strap.

4. Ultrasound system (1) according to one of the preceding claims,
**characterized in that**
the coupling between the sensor array (3) and the control unit (40) comprises a wire connection and/or a wireless interface.

5. Ultrasound system (1) according to one of the preceding claims,
**characterized in that**
a power source unit for providing energy to the transducer array (3) and/or the control unit (40).

6. Ultrasound system (1) according to one of the preceding claims,
**characterized in that,**
when the sleep mode is activated, the control unit (40) is configured to receive a wake-up signal and, subsequently, activate the measuring mode.

7. Ultrasound system (1) according to one of the preceding claims,
**characterized in that,**
when the sleep mode is activated, the standby frequency is set to a value between 25 and 50 kHz, preferably to a value of 32 kHz.

8. Ultrasound system (1) according to one of the preceding claims,
**characterized in that**
the sleep mode of the system is optimized such that the system's power consumption is minimized; wherein only modules that are needed for activating the measuring mode are kept running.

9. Ultrasound system (1) according to claim 8,
**characterized in that**
the modules, which are kept running, when the sleep mode is activated, comprise a wake-up controller module, an interrupt controller module, and/or a timer module.

10. Ultrasound system (1) according to one of the preceding claims,
**characterized in that**
the control unit (40) is configured, when activating the measuring mode after the sleep mode has been active, to perform a sleep-off sequence, wherein several modules of the system are subsequently activated.

11. Ultrasound system (1) according to claim 10,
**characterized in that**
during the power-on sequence, the first module to be activated comprises a phase-locked loop, PLL.

12. A method for operating an ultrasound system (1), wherein the ultrasound system has at least two operating modes, namely a sleep-mode and a measuring mode; wherein the ultrasound system operates such that, when the sleep-mode is activated, a low frequency system clock is running to a predetermined sleep frequency, and, when the measuring mode is activated, a high frequency system clock is set to a measuring frequency.

13. The method of claim 12,
**characterized in that**
the method is performed with the ultrasound system (1) according to one of claims 1 to 11.
